# EUROPEAN PATENT APPLICATION

(11) **EP 4 691 471 A1**
(43) Date of publication of application: **11.02.2026**
(21) Application number: 24193219.3
(22) Date of filing: 06.08.2024
(51) Int. Cl.: A61K 31/565, A61K 31/585, A61P 15/00, A61P 15/18

(54) **A COMPOSITION COMPRISING ESTETROL AND DROSPIRENONE FOR USE IN TREATING UNSCHEDULED BLEEDING IN ADOLESCENTS**

(71) Applicant: Estetra SRL, 4000 Liège (BE)
(72) Inventor: RUBAN, Kateryna, 4000 Liège (BE); CHATEL, Guillaume, 4000 Liège (BE); FLERIN, Nina, 4000 Liège (BE); GOBBI AMORIM, Fernanda, 4000 Liège (BE)
(74) Representative: De Clercq & Partners

(57) **Abstract**

The present invention relates to a composition comprising an estetrol component and drospirenone for use in treating unscheduled bleeding and spotting in an adolescent female subject. The invention also encompasses associated uses and methods of contraception in adolescent subjects.

## Description

### FIELD OF THE INVENTION

The present invention relates to compositions and methods for treating unscheduled bleeding in adolescent female subjects. Specifically, the invention relates to compositions comprising estetrol (E4) and drospirenone (DRSP), and associated methods that improve the bleeding profile.

### BACKGROUND OF THE INVENTION

Unscheduled bleeding is a prevalent and distressing issue among adolescent females, also among those using hormonal contraceptives. Associated with unscheduled bleeding are physical discomfort, psychological stress, and non-compliance with contraceptive regimens. Currently, treatment options for adolescent females are poorly understood, as most data come from studies involving adult populations. Recently, data from studies conducted specifically in the adolescent population became available for progestin-only pills based on drospirenone (e.g. SLYND^{®} (fda.gov)). Nevertheless, despite their advantages, progestin-only pills have the drawback of an unfavourable bleeding pattern and are hence not always preferred by premenopausal subjects. This suboptimal efficacy was even commented on and emphasized by the Food and Drug Administration (FDA) in a public letter to the manufacturer (FDA, Letter to Exeltis re NDA 211367 SLYND^{®} (drospirenone) tablets, for oral use, 2023).

Interestingly, there are notable discrepancies in the occurrence of unscheduled bleeding between adult and adolescent patients. While the percentage of adult females experiencing unscheduled bleeding/spotting with a progestin-only pill based on drospirenone was described to decrease over time, the percentage of adolescent females with unscheduled bleeding or spotting was maintained at a relatively consistent level during the study (53.0% in Cycle 1 versus 52.2% in Cycle 13, LABEL (fda.gov)).

Thus, adolescent users do not behave similarly. This discrepancy underscores the urgent need for novel therapeutic approaches tailored specifically to adolescents experiencing unscheduled bleeding. Addressing this unmet need, the present invention aims to provide a more effective, safe, and user-friendly solution for adolescent females seeking treatment for bleeding irregularities.

### SUMMARY OF THE INVENTION

Based on extensive research, the present inventors have shown that a composition comprising an estetrol component and drospirenone can be beneficial to adolescent subjects suffering from unscheduled bleeding and/or spotting.

A phase III study was conducted to assess safety, compliance and pharmacokinetics associated with the use of a COC comprising an estetrol component and drospirenone in post-menarchal adolescents. The COC was well-tolerated and no safety concerns were raised during the study. Bleeding data in adolescents showed good cycle control and acceptable bleeding pattern based on the gradual reduction in the incidence of unscheduled bleeding. The adolescents also experienced a reduction in the number of unscheduled bleeding and/or spotting days per cycle from 9 to 6 days. In addition, a mild and moderate improvement in respectively well-being and cycle-related symptoms was found.

Moreover, treatment in adolescents resulted in decreased dysmenorrhea symptoms, indicated by a decreased Visual Analogue Scale score >30% and a decreased use of rescue medication after three cycles of use with sustained relief up to the end of the study.

Notably, it was observed that a reduction in unscheduled bleeding and spotting occurred in a quick manner. This rapid decrease is unexpected, since the SLYND^{®} reported that the percentage of adolescent females with unscheduled bleeding or spotting was maintained at a relatively consistent level during treatment while the percentage of adult females experiencing unscheduled bleeding/spotting with SLYND^{®} decreased over time.

A further unexpected effect of the composition comprising an estetrol component and drospirenone was that despite the fact that having a stable bleeding pattern was not an inclusion requirement for the present study an excellent control of scheduled bleeding was observed, exceeding the scheduled bleeding control observed in adolescents upon use of SLYND^{®}, notwithstanding that the study using SLYND^{®} did require a stable bleeding pattern over a least four menstrual cycles prior to screening for enrolment eligibility.

Hence, the present invention provides for the following:
Aspect 1. A composition comprising an estetrol component and drospirenone for use in treating unscheduled bleeding and/or spotting in an adolescent female subject, preferably for decreasing unscheduled bleeding events and/or spotting events.
Aspect 2. A method of treating unscheduled bleeding and/or spotting in an adolescent female subject, comprising administering to said subject a composition comprising an estetrol component and drospirenone.
Aspect 3. A method of decreasing unscheduled bleeding events and/or spotting events in an adolescent female subject, comprising administering to said subject a composition comprising an estetrol component and drospirenone.
Aspect 4. Use of a composition comprising an estetrol component and drospirenone for the manufacture of a medicament for use in treating unscheduled bleeding and/or spotting in an adolescent female subject, preferably for decreasing unscheduled bleeding events and/or spotting events in an adolescent female subject.
Aspect 5. Use of a composition comprising an estetrol component and drospirenone for treating unscheduled bleeding and/or spotting in an adolescent female subject, preferably for decreasing unscheduled bleeding events and/or spotting events in an adolescent female subject.
Aspect 6. The composition according to aspect 1, the method according to aspect 2 or 3, or the use according to aspect 4 or 5, wherein a bleeding event is any evidence of vaginal blood loss that requires the use of sanitary protection with a tampon, menstrual cup, pad or pantyliner.
Aspect 7. The composition, method, or use according to any one of the preceding aspects, wherein a spotting event is any evidence of minimal vaginal blood loss that does not require new use of sanitary protection, including panty liners.
Aspect 8. The composition, method, or use according to any one of the preceding aspects, wherein an unscheduled bleeding event or spotting event is a bleeding or spotting event that is not considered a scheduled bleeding event or spotting event, preferably wherein a scheduled bleeding event or spotting event has:
   - a start date and stop date in the scheduled bleeding interval (day 25 - day 3 next cycle); or
   - a start date before day 25 and stop date before day 3 (early withdrawal bleeding/spotting); or
   - a start date in the scheduled bleeding interval (day 25 - day 3 next cycle) and continuing into next cycle (continued withdrawal bleeding/spotting).
Aspect 9. The composition, method, or use according to any one of the preceding aspects, wherein an unscheduled bleeding event or spotting event is a bleeding or spotting event that occurs outside of day 25 of a cycle to day 3 of a next cycle. In a preferred embodiment, an unscheduled bleeding event or spotting event is a bleeding or spotting event that has a start date outside day 25 to day 3 of a next cycle and/or a stop date outside day 25 to day 3 of a next cycle and/or a stop date as of day 3 of a next cycle.
Aspect 10. The composition, method, or use according to any one of the preceding aspects, wherein the first day of a cycle is defined as the day the subject uses or is administered the composition after at least 4 days, preferably at least 7 days, of no use or administration of said composition.
Aspect 11. The composition, method, or use according to any one of the preceding aspects, wherein the unscheduled bleeding events and/or spotting events are decreased compared to the unscheduled bleeding events and/or spotting events prior to the use of said composition.
Aspect 12. The composition, method, or use according to any one of the preceding aspects, wherein the decrease in the unscheduled bleeding events and/or spotting events prior to the use of the composition is a decrease in the unscheduled bleeding and/or spotting compared to the unscheduled bleeding events and/or spotting events upon using an oral contraceptive, preferably upon using a progestin-only pill preferably comprising drospirenone, preferably upon using a combined oral contraceptive comprising as estrogenic component a component that is not an estetrol component, preferably wherein said combined oral contraceptive comprising as estrogenic component a component that is not an estetrol component is a 2nd generation combined oral contraceptive.
Aspect 13. The composition, method, or use according to any one of the preceding aspects, wherein said unscheduled bleeding events and/or spotting events are decreased after 5 cycles, preferably after 4 cycles, preferably after 3 cycles, preferably after 2 cycles and most preferably after 1 cycle of administration.
Aspect 14. The composition, method, or use according to any one of the preceding aspects, wherein the decrease in unscheduled bleeding events and/or spotting events is a decrease in the number of occurrences of unscheduled bleeding events and/or spotting events, preferably over a period of at least one month.
Aspect 15. The composition, method, or use according to any one of the preceding aspects, wherein the unscheduled bleeding events and/or spotting events are decreased in occurrence and/or intensity by at least about 25%, preferably at least about 50%, more preferably by at least about 75%.
Aspect 16. The composition, method, or use according to any one of the preceding aspects, wherein the number of occurrences in unscheduled bleeding events and/or spotting events is decreased by at least about 25%, preferably at least about 50%, more preferably by at least about 75%.
Aspect 17. The composition, method, or use according to any one of the preceding aspects, wherein the number of days said unscheduled bleeding events and/or spotting events occur is reduced to 8 days or less, preferably to 6 days or less, more preferably 5 days or less within one cycle.
Aspect 18. The composition, method, or use according to any one of the preceding aspects, wherein the decrease in unscheduled bleeding events and/or spotting events is a decrease in the intensity of unscheduled bleeding events and/or spotting events, preferably over a period of at least one month.
Aspect 19. The composition, method, or use according to any one of the preceding aspects, wherein the intensity of unscheduled bleeding events and/or spotting events are decreased on average by at least about 25%, preferably at least about 50%, more preferably by at least about 75%.
Aspect 20. The composition, method, or use according to any one of the preceding aspects, wherein a bleeding event or spotting event corresponds to an episode of respectively vaginal bleeding or vaginal spotting, preferably wherein a vaginal bleeding episode or a vaginal spotting episode is respectively a vaginal bleeding event or a vaginal spotting event bordered on either end by at least 1 day of respectively no vaginal bleeding or vaginal spotting.
Aspect 21. The composition, method, or use according to any one of the preceding aspects, wherein the adolescent female subject is between 12 and 15 years old.
Aspect 22. The composition, method, or use according to any one of the preceding aspects, wherein the adolescent female subject is a postmenarchal subject.
Aspect 23. The composition, method, or use according to any one of the preceding aspects, wherein the adolescent female subject is a first-ever user, switcher, starter or restarter, preferably wherein said subject is a starter, preferably a starter that has not used hormonal contraceptives for 2 months before starting the use or administration of the composition, the method or the use according to any one of the preceding aspects.
Aspect 24. The composition, method, or use according to any one of the preceding aspects, wherein the estetrol component is administered at a daily dose of from about 1 mg to about 40 mg.
Aspect 25. The composition, method, or use according to any one of the preceding aspects, wherein the composition comprises from about 1 mg to about 40 mg of an estetrol component.
Aspect 26. The composition, method, or use according to any one of the preceding aspects, wherein the estetrol component is administered at a daily dose of from about 5 mg to about 25 mg, preferably at a daily dose of from about 10 mg to about 20 mg, more preferably at a daily dose of about 15 mg.
Aspect 27. The composition, method, or use according to any one of the preceding aspects, wherein the composition comprises from about 5 mg to about 25 mg of an estetrol component, preferably from about 10 mg to about 20 mg of an estetrol component, more preferably about 15 mg of an estetrol component, such as from 14 to 16 mg or 14.5 to 15.5 mg estetrol component.
Aspect 28. The composition, method, or use according to any one of the preceding aspects, wherein the drospirenone is administered at a daily dose of from about 0.5 mg to about 10 mg.
Aspect 29. The composition, method, or use according to any one of the preceding aspects, wherein the composition comprises from about 0.5 mg to about 10 mg of drospirenone.
Aspect 30. The composition, method, or use according to any one of the preceding aspects, wherein the drospirenone is administered at a daily dose of from about 1 mg to about 4 mg.
Aspect 31. The composition, method, or use according to any one of the preceding aspects, wherein the composition comprises from about 1 mg to about 4 mg of drospirenone.
Aspect 32. The composition, method, or use according to any one of the preceding aspects, wherein the method encompasses 21 to 28 daily administrations (i.e. a cyclical administration schedule), such as in cycles of 21, 22, 23, 24, 25, 26, 27, or 28 daily administrations by means of administration of a corresponding amount of daily active dosage units. The cycle preferably further comprises an administration-free interval of 7, 6, 5, or 4 days, more preferably an administration-free interval of about 4 days, such as between 3 and 5 days.
Aspect 33. The composition, method, or use according to any one of the preceding aspects, wherein the estetrol component is estretrol, preferably wherein the estetrol component is estetrol monohydrate.
Aspect 34. The composition, method, or use according to any one of the preceding aspects, wherein the estetrol component is administered at a daily dose of about 15 mg estetrol monohydrate, such as from 13 to 16 mg or 13.5 to 15.5 mg and preferably 14.2 mg estetrol monohydrate.
Aspect 35. The composition, method, or use according to any one of the preceding aspects, wherein the composition comprises about 15 mg of estetrol monohydrate, such as from 13 to 16 mg or 13.5 to 15.5 mg and preferably 14.2 mg estetrol monohydrate.
Aspect 36. The composition, method, or use according to any one of the preceding aspects, wherein the drospirenone is administered at a daily dose of about 3 mg, such as from 2.5 to 3.5 mg drospirenone.
Aspect 37. The composition, method, or use according to any one of the preceding aspects, wherein the composition comprises about 3 mg of drospirenone, such as from 2.5 to 3.5 mg drospirenone.
Aspect 38. The composition, method, or use according to any one of the preceding aspects, wherein the estetrol component and drospirenone are formulated together in a single oral dosage unit (i.e. a Combined Oral Contraceptive - COC).
Aspect 39. The composition, method, or use according to any one of the preceding aspects, wherein the composition, optionally the COC, is formulated to provide a daily dose of the estetrol component and drospirenone (i.e. formulated as a daily oral dosage unit).
Aspect 40. The composition, method, or use according to one of aspects 1 to 37, wherein the estetrol component is formulated in a first oral dosage unit and the drospirenone is formulated in a second oral dosage unit.
Aspect 41. The composition, method, or use according to aspect 40, wherein the first oral dosage unit is formulated to provide a daily dose of the estetrol component and the second oral dosage unit is formulated to provide a daily dose of the drospirenone.
Aspect 42. The composition, method, or use according to any one of the preceding aspects, wherein the subject has used the COC for at least one, two, three, or more cycles.

In any one of the aspects defined herein, said composition, optionally a COC, may be presented as a kit-of-parts containing a packaging unit, e.g. a blister pack, containing the daily oral dosage units comprising the estetrol component, preferably the estetrol, more preferably the estetrol monohydrate, and drospirenone. The skilled person will additionally know that, within the scope of the present invention, each packaging unit, e.g. blister pack, may be numbered or otherwise marked. In a particular embodiment of the kit-of-parts, the packaging unit comprises 28 containers or a multitude of 28 containers, such as 2 to 12 times 28 containers. In a preferred embodiment, the packaging unit comprises 3 or 6 times 28 containers. Within the scope of the invention, each packaging unit may be a sealed blister pack with a cardboard, paperboard, foil plastic backing and enclosed in a suitable cover.

Also envisaged in any one of the aspects defined herein are packaging units such as bottles. The material of the bottle is not particularly limiting. In preferred embodiments, the bottle is a glass bottle characterized by a colour capable of reducing or preventing degradation of the contents of the bottle by e.g. UV light while maintaining a degree of transparency that allows for visual inspection of the contents of said bottle. Suitable colours include without limitation amber, cobalt, or vintage green.

The above and further aspects and preferred embodiments of the invention are described in the following sections and in the appended claims. The subject matter of the appended claims is hereby specifically incorporated in this specification.

### BRIEF DESCRIPTION OF THE DRAWINGS

**Figure 1****:** Percentage of Subjects with Vaginal Bleeding and/or Spotting over Time (mITT Population, Evaluable Cycles only).
**Figure 2****:** Percentage of Subjects with Vaginal Bleeding and/or Spotting over Time (Per Protocol Population).
**Figure 3****:** Number and Percentage of Subjects with scheduled or unscheduled Vaginal Bleeding and/or Spotting or Absence of any Vaginal Bleeding and/or Spotting over Time (mITT Population).
**Figure 4****:** Incidence of Unscheduled Bleeding and/or Spotting Over Time by Treatment Cycle (mITT Population, Evaluable Cycles only). The incidence of unscheduled bleeding/spotting is reduced after the first cycle and consists mostly of spotting episodes.

### DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of', which enjoy well-established meanings in patent terminology.

The recitation of numerical ranges by endpoints includes all numbers and fractions subsumed within the respective ranges, as well as the recited endpoints. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g. any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e. also in the context of other aspects or embodiments of the invention, unless otherwise defined. For example, embodiments directed to products are also applicable to corresponding features of methods and uses.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, alternative combinations of claimed embodiments are encompassed, as would be understood by those in the art.

Unless indicated otherwise, all methods, steps, techniques and manipulations that are not specifically described in detail can be performed and have been performed in a manner known per se, as will be clear to the skilled person. Reference is for example again made to standard handbooks as well as to the general background art referred to herein and to the further references cited therein.

The term "Combined Oral Contraceptives" (COCs), as used throughout the present specification, represent a class of pharmaceutical formulations aiming to provide a comprehensive and reliable method for preventing pregnancy through oral administration. The synergistic action of an estrogenic and a progestogenic component mimics the physiological intricacies of the menstrual cycle, exerting multifaceted contraceptive effects. Particularly, COCs suppress ovulation. In addition, COCs create cervical mucus alterations, hindering sperm penetration into the uterus, and induce modifications in the endometrial lining, rendering it less receptive to embryo implantation. COCs therefore provide a high degree of contraceptive efficacy and other benefits such as the regulation of menstrual cycles and reduction of menstrual pain.

The term "estetrol" as used herein refers to 1,3,5 (10)-estratrien-3,15alpha,16alpha,17beta-tetrol or 15alpha-hydroxyestriol as well as hydrates of estetrol, e.g. estetrol monohydrate. "Estetrol", or short "E4" is an estrogen steroid produced by the foetal human liver (PubChem CID: 27125). Estetrol may be described as a 3-hydroxy steroid corresponding to 17beta-estradiol wherein the 15α and 16α positions are substituted for two additional hydroxy groups. It is known that estetrol is an estrogen receptor agonist (Coelingh Bennink et al., Climacteric, 2008). The estetrol may be chemically synthetised, synthesised by the use of (mutant) recombinant enzymes, or synthesised by any combination thereof. It is therefore evident that the terms "estetrol" and "estetrol components" equally encompass further chemically modified estetrol. Estetrol may be indicated in the art by its molecular formula: C₁₈H₂₄O₄, or by structural formula (I).

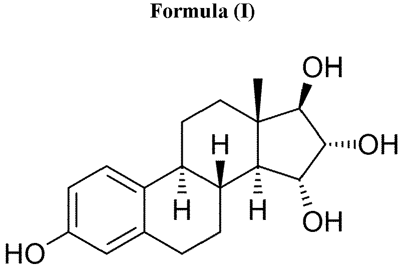

It is understood that when estetrol is mentioned throughout any section of this specification, any estetrol-containing component (i.e. compound) and/or estetrol derivative (such as an estetrol ester) is also envisaged. More preferably, in the context of the present disclosure, a particularly preferred estetrol (component) is estetrol monohydrate. Estetrol monohydrate is a white to off-white crystalline solid that is poorly soluble in water and aqueous solutions. It is soluble in methanol, ethanol, sparingly soluble in acetone, and slightly soluble in ethyl acetate and acetonitrile. A skilled person appreciates that estetrol monohydrate corresponds to estetrol containing one molecule of water, and that the core structural formula of estetrol does not differ from Formula (I). By means of illustration and not limitation, the structural formula of estetrol monohydrate is indicated by Formula (II):

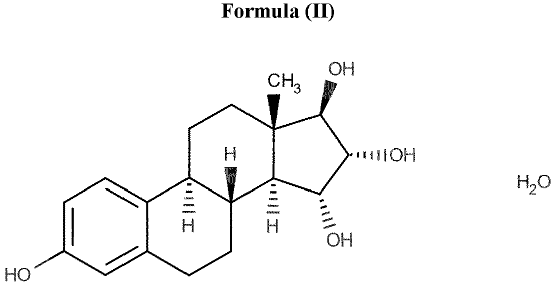

"Drospirenone" (abbreviated as DRSP, PubChem CID: 68873) is an example of a progestogenic component and enjoys a widespread use in Combined Oral Contraceptives (commonly abbreviated as COCs) due to its antimineralocorticoid and antiandrogenic activity combined with a general low off-target activity. Drospirenone is a white to almost white or slightly yellow crystalline powder. It is a neutral molecule with slight solubility in water. In general, drospirenone-containing COCs are referred to as fourth generation COCs. Non-limiting examples of commercially available COCs comprising drospirenone are known as "Yaz^{™}" and Yasmin^{™}. An illustrative example of a drospirenone only progestogen pill is "Slynd^{™}", which is also commercially available. Additionally, hormone replacement therapy compositions comprising an estrogen such as estradiol and drospirenone are available such as "Angeliq^{™}". Drospirenone may alternatively be indicated in the art by its molecular formula C₂₄H₃₀O₃, or by the structural formula (III):

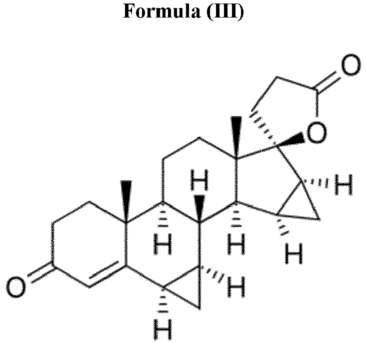

It is understood that when the term "drospirenone" is used herein, any drospirenone derivatives are also envisaged. The terms "progestogen", "gestagen", or "gestogen" and derived hereof "progestogenic components" as used both herein and in the art refer to any molecule that produces effects similar to those of the natural female sex hormone progesterone in the body of a subject. Progestogens are considered to be agonists of the progesterone receptors and their functions have been thoroughly examined in the art (inter alia discussed in Kuhl, Climacteric, 2005). Progestins are a subgroup of progestogens that comprise synthetic progestogens. While the above terms may be used interchangeably in the art, there is a general understanding that when progestin is mentioned, synthetic progestogens are meant.

Numerous references are made throughout the present specification to indicate that a certain condition in a subject is changed, i.e. treated. The terms "treatment" or "treat" are to be interpreted as both the therapeutic treatment of a symptom, disease or condition that has already developed, leading to (clinical) manifestations, as well as prophylactic or preventive measures, wherein the goal of the treatment is to prevent, lessen, or reduce the chances of incidence of an undesired affliction, such as to prevent occurrence, development and progression of blood pressure increases and/or hypertension, and (clinical) conditions that are a consequence thereof.

"Prevention" or "prevent" as used in the context of the invention refers to an aversion of manifestation of a condition or disease image in a subject, i.e. the establishment of preventive measures or prophylactic measures. Preventive treatment refers to treatments wherein the object is to avoid a subject's body or an element thereof to show (worsening of) symptoms of an undesired physiological or psychological change, in the present context for example an increase of blood pressure. As used herein, the term "prevent" includes both preventing symptoms from occurring and preventing symptoms of worsening.

Unexpectedly, the inventors have found that a composition comprising an estetrol component and drospirenone has beneficial effects upon use in adolescents when compared to other hormone-based contraceptives for which data are available. More particularly, remarkable effects were observed with respect to a sharp decrease in unscheduled bleeding events about immediately upon the start of treatment, and a scheduled bleeding cycle control that surpasses that of other contraceptives for which data are available. In addition, administration of the composition as described herein resulted in an improvement in both well-being and cycle-related symptoms, as well a decrease in dysmenorrhea symptoms.

As outlined below in the Examples, a Phase III study was conducted to assess safety, compliance and pharmacokinetics associated with the use of the composition disclosed herein in post-menarchal adolescents of 12 to 17 years for six cycles. The mean age of the study was 15.2 years old and 91.4% of the 105 included participants were starters.

The composition disclosed herein was well-tolerated and no safety concerns were raised during the study. Bleeding data in adolescents showed good cycle control and acceptable bleeding pattern based on the gradual reduction in the incidence of unscheduled bleeding from 45.8% at cycle 1 to 14.5% at cycle 5 and were consistent with those from Phase III Studies in adult females, contrary to what was reported for progestin only pills.

The adolescents also experienced a reduction in the number of unscheduled bleeding and/or spotting days per cycle from 9 to 6 days. For cycles 1 to 5, the incidence of scheduled bleeding ranged between 77.4% and 90.5%, indicating predictability of vaginal bleeding. KIDSCREEN-27 and MDQ scores indicated mild and moderate improvement, respectively, in well-being and cycle-related symptoms.

The KIDSCREEN-27 is a generic self-report measure to assess health-related quality of life for children and adolescents. The KIDSCREEN measure is applicable for healthy and chronically ill children and adolescents from 8 to 18 years. It can be administrated in hospitals, medical establishments, and schools by professionals in the fields of Public Health, Epidemiology, and Medicine. The KIDSCREEN instruments assess children's and adolescents' subjective health and well-being (health-related quality of life, HRQOL). (The KIDSCREEN Instruments, 2004; The KIDSCREEN Questionnaires, 2006).

The MDQ is a standard method for measuring cyclical perimenstrual symptoms. The MDQ is a 46-item self-report inventory for use in the assessment and treatment of premenstrual and menstrual symptoms. The MDQ can distinguish cyclical from noncyclical changes in physical symptoms, mood and behavior, and arousal. It identifies the type and intensity of symptoms women experience during each phase of the menstrual cycle and can aid researchers and clinicians in specifying the effect of therapeutic interventions.

Additionally, treatment with the composition disclosed herein in adolescents resulted in decreased dysmenorrhea symptoms, indicated by a decreased Visual Analogue Scale score >30% and a decreased use of rescue medication after three cycles of use with sustained relief up to the end of the study.

Accordingly, a first aspect of the invention is directed to a composition comprising an estetrol component and drospirenone for use in treating unscheduled bleeding and/or spotting in an adolescent female subject. Alternatively worded, the invention provides a method of treating unscheduled bleeding and/or spotting in an adolescent female subject, comprising administering to said subject a composition comprising and estetrol component and drospirenone. Equally envisaged is the use of a composition comprising an estetrol component and drospirenone for the manufacture of a medicament for use in treating unscheduled bleeding and/or spotting in an adolescent female subject, and the use of a composition comprising an estetrol component and drospirenone for treating unscheduled bleeding and/or spotting in an adolescent female subject.

A skilled person understands that the commonly accepted meaning of the term "unscheduled bleeding and/or spotting" particularly in adolescents using contraceptives, refers to any vaginal bleeding that occurs outside of the expected menstrual period or the regular withdrawal bleeding associated with contraceptive use. This can include: breakthrough bleeding, i.e. bleeding that occurs between menstrual periods; spotting, i.e. light bleeding that doesn't require sanitary protection; and/or prolonged bleeding, i.e. bleeding episodes lasting longer than usual; Frequent bleeding, i.e. more than five bleeding episodes in a cycle. Spotting can be rather common, especially in the first few months of starting a new hormonal contraceptive method. It often resolves on its own, but if it persists, it's important to consult a healthcare provider to rule out any underlying issues. Unless specifically indicated otherwise, in the present context a bleeding event is considered any evidence of vaginal blood loss that requires the use of sanitary protection. Suitable sanitary protection includes but is not limited to a tampon, menstrual cup, pad or pantyliner. Unless specifically indicated otherwise, in the present context a spotting event is any evidence of minimal vaginal blood loss that does not require new use of sanitary protection, including panty liners.

A skilled person readily appreciates that an unscheduled bleeding event or spotting event is a bleeding or spotting event that is not considered a scheduled bleeding event or spotting event. Preferably, a scheduled bleeding event or spotting event has:
- a start date and stop date in the scheduled bleeding interval (day 25 - day 3 next cycle); or
- a start date before day 25 and stop date before day 3 (early withdrawal bleeding/spotting); or
- a start date in the scheduled bleeding interval (day 25 - day 3 next cycle) and continuing into next cycle (continued withdrawal bleeding/spotting).

Preferably, an unscheduled bleeding event or spotting event is a bleeding or spotting event that occurs outside of day 25 of a cycle to day 3 of a next cycle. Preferably, an unscheduled bleeding event or spotting event is a bleeding or spotting event that has a start date outside day 25 to day 3 of a next cycle and/or a stop date outside day 25 to day 3 of a next cycle and/or a stop date as of day 3 of a next cycle. It is further understood that a bleeding event or spotting event corresponds to an episode of respectively vaginal bleeding or vaginal spotting bordered on either end by at least 1 day of respectively no vaginal bleeding or vaginal spotting. Optionally, the bleeding event or spotting event corresponds to an episode of respectively vaginal bleeding or vaginal spotting bordered on either end by at least 2 days of respectively no vaginal bleeding or vaginal spotting.

A skilled person appreciates that the first day of a cycle is defined as the day the subject uses or is administered the composition after at least 4 days, preferably at least 7 days, of no use or administration of the composition.

As indicated above, administration or use of the composition subject of the present disclosure leads to a reduction in unscheduled bleeding events and/or spotting events compared to the unscheduled bleeding events and/or spotting events prior to the use of said composition. The reduction may be a reduction in frequency (i.e. occurrence), intensity, or a combination thereof. Alternatively, the reduction may be a decrease in the number of occurrences without any substantial decrease in the intensity of the unscheduled bleeding events and/or spotting events, or a decrease in the intensity without any substantial decrease in the number of occurrences of the unscheduled bleeding events and/or spotting events. Preferably, the decrease is a decrease in the number of occurrences and a decrease in the intensity of the unscheduled bleeding events and/or spotting events. "Occurrence" as used herein is to be interpreted as an event that extends over a certain period without a break. Preferably, a break in the present context is to be interpreted as an interruption of at least 12, preferably 24 hours. Bleeding or spotting after the interruption of at least 12, preferably 24 hours should be considered a new (i.e. different, further) occurrence.

References throughout the present disclosure such as "prior to the use of the composition" and interchangeably usable expressions refer to a point in time wherein the subject was not subjected to the composition subject of the present disclosure. Said point in time may relate to a point in time where the subject was not administered or using any hormone-based medication, or any estrogen-containing medication. Alternatively, said point in time may relate to a time wherein the subject was administered or using a contraceptive, such as an oral contraceptive. Preferably, said point in time may relate to a time wherein the subject was administered or using an oral contraceptive such as a combined oral contraceptive (COC) or a progestogen-only contraceptive (such as but not limited to a drospirenone-only pill). Preferably, said point in time relates to a time wherein the subject was administered or using a combined oral contraceptive (COC) comprising an estrogenic component that is not an estetrol component. By means of illustration and not limitation a COC comprising an estrogenic component that is not an estetrol component may relate to a 2^{nd} generation COC. A skilled person readily appreciates that a 1st generation COC refers to COCs that contain estrogens other than ethinylestradiol (EE) combined with progestogens such as norethisterone and norethindrone, 2nd generation COCs are COCs combined ethinylestradiol with progestogens derived from testosterone (e.g. levonorgestrel (LNG) and norgestrel). 3rd generation COCs contain progestogens derived from levonorgestrel (e.g. gestodene, desogestrel, norgestimate), and 4th generation COC are COCs containing anti-androgenic progestogens such as cyproterone acetate, drospirenone (DRSP), dienogest and chlormadinone.

While the inventors have found that the composition subject of the present disclosure elicits its effects in a substantially swift manner, the timing (i.e. onset) of the unscheduled bleeding events and/or spotting events may vary according to different response timings when comparing different subjects. Preferably, the unscheduled bleeding events and/or spotting events are decreased after 5 cycles, more preferably after 4 cycles, more preferably after 3 cycles, more preferably after 2 cycles, and most preferably after 1 cycle of administration of the composition. The decrease in occurrences and/or intensity of unscheduled bleeding events and/or spotting events may be evaluated by any suitable means and over any suitable time period. Preferably, the decrease is an decrease over a period of at least one month, optionally 2 months, optionally 3 months, or optionally 6 months.

In certain embodiments, the unscheduled bleeding events and/or spotting events are decreased in occurrence and/or intensity by at least about 10%, preferably by at least about 25%, preferably at least about 50%, more preferably by at least about 75% when compared to a point in time wherein the subject was not administered or using any contraceptive or was administered or using a COC having as estrogenic component an estrogen that is not estetrol, more preferably a COC having ethinylestradiol as estrogenic component. Thus, optionally the number of occurrences in unscheduled bleeding events and/or spotting events is decreased by at least about 10%, preferably by at least about 25%, preferably at least about 50%, more preferably by at least about 75% when compared to a point in time wherein the subject was not administered or using any contraceptive or was administered or using a COC having as estrogenic component an estrogen that is not estetrol, more preferably a COC having ethinylestradiol as estrogenic component. Optionally the intensity of unscheduled bleeding events and/or spotting events is decreased by at least about 10%, preferably by at least about 25%, preferably at least about 50%, more preferably by at least about 75% when compared to a point in time wherein the subject was not administered or using any contraceptive or was administered or using a COC having as estrogenic component an estrogen that is not estetrol, more preferably a COC having ethinylestradiol as estrogenic component.

Optionally, the number of days said unscheduled bleeding events and/or spotting events occur is reduced to 8 days or less, preferably 7 days or less, preferably to 6 days or less, preferably 5 days or less, preferably 4 days or less, preferably 3 days or less, preferably 2 days or less, preferably 1 day, or even reduced to 0 days within one cycle.

The terms "selected" or "selection", "diagnosed" or "diagnosis, "predicted" or "prediction" and, "prognosticated" or "prognosis" may be used interchangeably herein and are commonplace and well-understood in medical and clinical practice. It shall be understood that the phrase "a method for the diagnosis, prediction and/or prognosis" a given disease or condition may also be interchanged with phrases such as "a method for diagnosing, predicting and/or prognosticating" of said disease or condition or "a method for making (or determining or establishing) the diagnosis, prediction and/or prognosis" of said disease or condition, or the like.

Without limitation, the terms "predicting" or "prediction" generally refer to a statement, declaration, indication or foretelling of a disease or condition in a subject not (yet) showing any, or a limited, clinical manifestation of said disease or condition. A prediction of a disease or condition in a subject may indicate a probability, chance or risk that the subject will develop said disease or condition, for example within a certain time period or by a certain age. Said probability, chance or risk may be indicated as any suitable qualitative or quantitative expression, wherein non-limiting examples of a quantitative expression include absolute values, ranges or statistics. Alternatively the probability, chance, or risk may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. The term "prediction" of the conditions or diseases as taught herein in a subject may also particularly mean that the subject has a 'positive' prediction of such, i.e., that the subject is at risk of having such (e.g., the risk is significantly increased vis-à-vis a control subject or subject population).

The terms "diagnosing" or "diagnosis" are indicative for a process of recognizing, deciding on or concluding on a disease or condition in a subject on the basis of symptoms and signs and/or from results of various diagnostic procedures (such as, for example, from knowing the presence, absence and/or quantity of one or more biomarkers of or clinical symptoms characteristic for the diagnosed disease or condition). "Diagnosis of" the diseases or conditions as taught herein in a subject may particularly mean that the subject has such disease or condition. A subject may be diagnosed as not having such despite displaying one or more conventional symptoms or signs reminiscent of such.

In view of the present invention, the female subject typically is an adolescent subject. Adolescence is defined as the transitional period of growth and development between childhood and adulthood. The age range can vary from subject to subject, and runs from the onset of puberty (including the onset of menstruation or menarche and the postmenarche age) to the attainment of full adult maturity. The World Health Organization (WHO) defines adolescence as the age range from 10 to 19 years, however, some definitions extend this period to up to 24 years to include late adolescence and the transition to adulthood. The term "adolescence" in the light of the present invention includes the broader age range of 10 to 24 years. Hence, typically, the adolescent subject is between 10 and 24 years, such as between 10 and 23 years, between 10 and 22 years, between 10 and 21 years, between 10 and 20 years, or between 10 and 19 years old. More particularly, the subject can be between 11 and 18 years, or between 12 and 17 years old, more particular between 10 and 16 years, between 11 and 16 years, between 12 and 16 years, between 10 and 15 years, between 11 and 15 years, or between 12 and 15 years old.

The term postmenarchal refers to the period after a female has experienced her first menstrual cycle, known as menarche. This term is often used in medical and developmental contexts to describe girls or women who have begun menstruating.

A skilled person is aware that terms such as "quantity", "amount" and "level" are synonyms and have a well-defined meaning in the art. The terms as used herein may particularly refer to an absolute quantification of a molecule such as a steroid, in (a sample taken from) a subject, or to a relative quantification of a molecule or analyte in a sample, i.e., relative to another value such as relative to a reference value as taught herein, or to a range of values indicating a base-line of a certain parameter. These values or ranges of values may be obtained from one single subject or from a group of subjects (i.e. at least two subjects).

As detailed above, the composition described herein is typically administered at a daily amount of from about 1 mg to about 40 mg of estetrol, estetrol monohydrate, or an ester of estetrol. Evidently, this includes embodiments wherein the composition described herein comprises from about 1 mg to about 40 mg of estetrol, estetrol monohydrate, or an ester of estetrol (a skilled person appreciates that this is encompassed by the definition of "equivalent to from about 1 mg to about 40 mg of estetrol"). In further embodiments, the composition described herein is administered at a daily amount of from about 5 mg to about 25 mg of estetrol, estetrol monohydrate, or an ester of estetrol, or comprises from about 5 mg to about 25 mg of estetrol, estetrol monohydrate, or an ester of estetrol. Preferably the composition the composition described herein is administered at a daily amount of from about 10 mg to about 20 mg of estetrol, estetrol monohydrate, or an ester of estetrol, or comprises from about 10 mg to about 20 mg of estetrol, estetrol monohydrate, or an ester of estetrol. In further embodiments, the composition described herein is administered at a daily amount of about 15 mg of estetrol, estetrol monohydrate, or an ester of estetrol, or comprises about 15 mg (such as 14 to 16 mg) of estetrol, estetrol monohydrate, or an ester of estetrol. In a preferred embodiment, the composition is administered at a daily amount of 14.2 mg of estetrol or comprises 14.2 mg of estetrol. In alternative embodiments, the composition described herein is administered at a daily amount of about 20 mg (such as 19 to 21 mg) of estetrol, estetrol monohydrate, or an ester of estetrol, or comprises about 20 mg of estetrol, estetrol monohydrate.

In preferred embodiments, the composition described herein is administered at a daily amount of from about 0.5 mg to about 10 mg of drospirenone. Evidently, this includes embodiments wherein the composition comprises from about 0.5 mg to about 10 mg of drospirenone (a skilled person appreciates that this is encompassed by the definition of "equivalent to from about 0.5 mg to about 10 mg of drospirenone"). In further embodiments, the composition described herein is administered at a daily amount of from about 1 mg to about 4 mg of drospirenone, or comprises from about 1 mg to about 4 mg of drospirenone. In further embodiments, the composition is administered at a daily amount of about 3 mg of drospirenone, or comprises about 3 mg (such as 2.5 to 3.5) of drospirenone.

In yet further preferred embodiments, the composition described herein is administered at a daily amount of from about 1 mg to about 40 mg of estetrol, estetrol monohydrate, or an ester of estetrol and at a daily amount of from about 0.5 mg to about 10 mg of drospirenone. In further embodiments, the composition described herein comprises from about 1 mg to 40 mg of estetrol, estetrol monohydrate, or an ester of estetrol and from about 0.5 mg to about 10 mg of drospirenone. Preferably, the composition described herein is administered at a daily amount of from about 5 mg to about 25 mg of estetrol, estetrol monohydrate, or an ester of estetrol and at a daily amount of from about 1 mg to about 4 mg of drospirenone. In further embodiments, the composition described herein comprises from about 5 mg to 25 mg of estetrol, estetrol monohydrate, or an ester of estetrol and from about 1 mg to about 4 mg of drospirenone. Yet more preferably, the composition described herein is administered at a daily amount of from about 10 mg to about 20 mg of estetrol, estetrol monohydrate, or an ester of estetrol and at a daily amount of from about 1 mg to about 4 mg of drospirenone. In further embodiments, the composition described herein comprises from about 10 mg to 20 mg of estetrol, estetrol monohydrate, or an ester of estetrol and from about 1 mg to about 4 mg of drospirenone. Most preferably, the composition described herein is administered at a daily amount of about 15 mg of estetrol, estetrol monohydrate, or an ester of estetrol and at a daily amount of about 3 mg of drospirenone. In further embodiments, the composition described herein comprises about 15 mg (such as 14 to 16 mg) of estetrol, estetrol monohydrate, or an ester of estetrol and about 3 mg (such as 2.5 to 3.5 mg) of drospirenone. In preferred embodiments, the composition described herein comprises 14.2 mg of estetrol and 3 mg of drospirenone.

Optionally, the composition described herein comprises estetrol monohydrate administered in a daily amount of from about 1 mg to about 40 mg and drospirenone administered in a daily amount of from about 0.5 mg to about 10 mg. In further optional embodiments, the composition described herein is administered in a daily amount of from about 5 mg to about 25 mg of estetrol monohydrate, and drospirenone administered in a daily amount of from about 1 mg to about 4 mg. In further optional embodiments, the composition described herein is administered in a daily amount of from about 10 mg to about 20 mg of estetrol monohydrate, and drospirenone administered in a daily amount of from about 1 mg to about 4 mg. In yet further optional embodiments, the composition described herein is administered in a daily amount of about 15 mg (such as 14 to 16 mg) estetrol monohydrate, and drospirenone administered in a daily amount of about 3 mg (such as 2.5 to 3.5 mg). In preferred embodiments, the composition described herein is administered in a daily amount of 14.2 mg estetrol, and drospirenone administered in a daily amount of about 3 mg.

In certain embodiments, the composition described herein comprises estetrol, preferably estetrol monohydrate, in an amount from about 1 mg to about 40 mg and drospirenone in an amount of from about 0.5 mg to about 10 mg. In further optional embodiments, the composition described herein comprises from about 5 mg to about 25 mg of estetrol monohydrate, and from about 1 mg to about 4 mg drospirenone. In further optional embodiments, the composition described herein comprises from about 10 mg to about 20 mg of estetrol monohydrate, and from about 1 mg to about 4 mg drospirenone. In yet further optional embodiments, the composition described herein comprises about 15 mg (such as 14 to 16 mg) estetrol monohydrate, and about 3 mg (such as 2.5 to 3.5 mg) of drospirenone.

Preferably, the composition described herein is used in cycles of from 21 to 28 daily administrations (i.e. a cyclical administration schedule). The composition described herein may be used in cycles of 21, 22, 23, 24, 25, 26, 27, or 28 daily administrations by means of administration of a corresponding amount of daily active dosage units. The cycle preferably further comprises an administration-free interval of 7, 6, 5, or 4 days. Preferably, the cycle comprises an administration-free interval of 4 days. The number of cycles the COC subject of the invention may be used is not particularly limiting for the invention. Hence, the composition described herein may be used for at least 1, 2, 3, 4, 5, 6, 7, 8, 9, 10, 11, 12, 13 or more than 13 cycles. The favourable effect on the blood pressure of the subject may be apparent after using the COC for 1, 2, 3, 4, 5, 6, 7, 8, 9, 10, 11, 12, 13, or more than 13 cycles.

The composition described herein may be comprised of a single dosage unit, i.e. a dosage unit wherein the estetrol and drospirenone are contained in the same dosage unit, said dosage unit corresponding to the composition described herein that is to be administered to the subject. Alternatively, the composition described herein may be comprised of at least two dosage units wherein one dosage unit comprises the estetrol and a second dosage unit comprises the drospirenone. In such embodiments, the composition described herein may be a two-part composition described herein comprising two separate dosage units conjoined to one another. In preferred embodiments, the composition described herein is formulated to provide a daily dose of the estetrol and the drospirenone (i.e., the composition described herein is formulated as a daily oral dosage unit). "Dosage unit", interchangeably used with "dosage form" herein and in the art indicates a physical preparate that is suitable for administration to a subject, without the necessity to adapt the drug product prior to administration, i.e. the final beneficial product. A dosage unit therefore indicates a ready-to-administer composition (in the present context a ready-to administer composition). The term is not limiting for any other particulars of the treatment, such as frequency of administration and/or any characteristic of the dosage unit (taste, appearance, size, etc.).

Optionally, the composition described herein is formulated as a daily dosage unit. Optionally, the composition described herein is formulated as an oral dosage unit. The composition described herein is particularly suited for formulation as an oral dosage unit with oral ingestion as the envisaged mode of administration. After oral ingestion, the active ingredient is subjected to the first-pass effect. However, equally envisaged are dosage units formulated towards alternative administration methods such as but not limited to sublingual, buccal, or sublabial dosage units, that are intended to avoid the first-pass effect. The (solid) composition described herein may therefore be suitably or even specifically manufactured for sublingual, buccal, and/or sublabial administration. The dosage unit may be able to rapidly release the estetrol and drospirenone when contacted with an aqueous solvent such as saliva. Hence, in such embodiments the composition described herein is an orodispersible composition which releases at least about 50%, preferably at least about 60%, more preferably at least about 70%, yet more preferably at least about 80%, most preferably more than about 80% of the estetrol and/or drospirenone within about 5 minutes, preferably within about 3 minutes, more preferably within about 2.5 minutes, more preferably within about 90 seconds, most preferably within about 90 seconds. In further embodiments, the composition described herein rapidly disintegrates in the oral cavity when it comes into contact with saliva and disperses the estetrol and/or drospirenone into the saliva so it may be absorbed through the mucosal lining of the oral cavity. A skilled person is aware of methods to determine the release rate of a pharmaceutically active ingredient such as estetrol and drospirenone from a dosage unit. Non-limiting standardized tests generally accepted in the field include the disintegration test according to Ph. Eur. 2.9.1 ("Disintegration of tablets and capsules") and USP <701> ("Disintegration"), for example using water as the disintegration medium.

The term "sublingual" as used herein refers to the pharmacological route of administration by which the estetrol and/or drospirenone (comprised in the composition) diffuse into the blood through tissues under the tongue.

The term "buccal" as used herein refers to the pharmacological route of administration by which the estetrol and/or drospirenone (comprised in the composition) diffuse into the blood through tissues of the buccal vestibule, the area inside the mouth between the lining of cheek (the buccal mucosa) and the teeth / gums.

The term "sublabial" as used herein refers to the pharmacological route of administration by which the estetrol and/or drospirenone (comprised in the composition) are placed between the lip and the gingiva.

Optionally, the dosage unit can be administered to a subject by means of a continuous administration schedule. The terms "continuous" and "continuously" as used herein, means that the dosage units are administered at relatively regular intervals, with no (therapeutically) significant interruptions. Naturally, minor interruptions may occur that do not affect the overall effectiveness of the present method, and indeed such aberrations are encompassed by the present invention. Preferably and more arithmetically, the administration regimen is deemed to be continuous if the longest interval between two subsequent administrations is not more than 3.5 times as long as the average interval. Even more preferably said longest interval is not more than 2.5 times, most preferably not more than 1.5 times as long as the average interval. By means of illustration and not limitation, the treatment strategies and method of treatments described herein preferably employ continuous administration of the estetrol and drospirenone during a period of at least 10 days, preferably of at least 20 days.

Alternatively, the dosage unit can be administered to a subject by means of a sequential administration schedule. It is to be appreciated that the term "sequential" means an administration during, for example, 10 to 14 days each month or during 14 days every 3 months. Sequential administration schedules are particularly envisaged wherein a progestogenic component is part of the composition or treatment described herein.

It is evident that any of the compositions and dosage units described herein may suitably contain one or more pharmaceutically acceptable excipients. The term "pharmaceutically acceptable" as used herein is consistent with the art and means compatible with the other ingredients of a pharmaceutical composition and not deleterious to the recipient thereof.

As described above, the composition as described herein, and hence the (oral) dosage unit may comprise one or more suitable excipients. The term "excipient" may be a "carrier" indicative for any solvent, diluent, buffer (including but not limited to neutral buffered saline, phosphate buffered saline, or optionally Tris-HCl, acetate or phosphate buffers), solubiliser (including but not limited to Tween 80 or Polysorbate 80), colloid, dispersion medium, vehicle, filler, chelating agent (including but not limited to EDTA or glutathione), amino acid, protein, disintegrant, binder, lubricant, wetting agent, stabiliser, emulsifier, sweetener, colorant, flavoring, aromatiser, thickener, any agent suitable to achieve a depot effect, coating, antifungal agent, any preservative (including but not limited to Thimerosal^{™} or thiomersal, benzalkonium chloride, or benzyl alcohol), antioxidant (including but not limited to ascorbic acid, sodium metabisulfite), tonicity controlling agent, absorption delaying agent, adjuvant, bulking agent (including but not limited to lactose, mannitol) and any other ingredient that may influence any parameter or characteristic of the oral dosage unit subject of the invention. A skilled person understands that one or more excipients may be used in the composition or oral dosage unit on condition that the one or more excipient is compatible with the pharmaceutical ingredients (i.e. in the context of the present disclosure at least estetrol and drospirenone) and that a pharmaceutically acceptable formulation is obtained.

In certain embodiments, said excipient may be an active pharmaceutical ingredient excipient, binder excipient, carrier excipient, co-processed excipient, coating system excipient, controlled release excipient, diluent excipient, disintegrant excipient, dry powder inhalation excipient, effervescent system excipient, emulsifier excipient, lipid excipient, lubricant excipient, modified release excipient, penetration enhancer excipient, permeation enhancer excipient, pH modifier excipient, plasticiser excipient, preservative excipient, preservative excipient, solubiliser excipient, solvent excipient, sustained release excipient, sweetener excipient, taste making excipient, thickener excipient, viscosity modifier excipient, filler excipient, compaction excipient, dry granulation excipient, hot melt extrusion excipient, wet granulation excipient, rapid release agent excipient, increased bioavailability excipient, dispersion excipient, solubility enhancement excipient, stabilizer excipient, capsule filling excipient, or any combination hereof. A skilled person is aware that use of such media and agents for pharmaceutical active substances is common practice and incorporation of these excipients is hence well known in the art. It is evident that all of the used ingredients should be non-toxic in the concentration contained in the final pharmaceutical composition and should not negatively interfere with the activity of the one or more pharmaceutically active ingredients, in this context at least the estetrol component.

The composition described herein may alternatively be described as a solid or semi solid dosage unit such as a tablet, a capsule, a cachet, a pellet, a pill, powder, granules, or any combination thereof. For example, the composition described herein may be a tablet comprising estetrol-containing granules and drospirenone or a capsule comprising estetrol-containing granules and drospirenone. Optionally, the drospirenone may be comprised in the estetrol-containing granules. Alternatively, the drospirenone may be comprised in distinct granules, i.e. granules that do not comprise estetrol. The term "solid or semi-solid dosage unit" also encompasses capsules that contain a liquid, e.g. an oil, in which estetrol and drospirenone are dissolved or dispersed.

Tablets and equivalent solid and semi-solid dosage units can suitably contain materials such as binders (e.g. hydroxypropylmethyl cellulose, polyvinyl pyrrolidone (povidone, PVP), other cellulosic materials and starch), diluents (e.g. lactose (monohydrate) and other sugars, starch (e.g. maize starch), dicalcium phosphate and cellulosic materials), disintegrating agents (e.g. starch polymers and cellulosic materials (e.g. sodium starch glycolate) and lubricating agents (e.g., (magnesium) stearates and talc). These tablets and equivalent solid dosage units may be prepared by any suitable means, which have been described in detail in the art (e.g. Kaur, Int Res J Pharm, 2012). Non-limiting examples of processing methods of the estetrol and drospirenone when manufacturing the dosage unit include wet granulation, e.g. using an aqueous solution or an organic solution, direct compression, 3D printing, or by coating carrier particles with the estetrol and optionally with the drospirenone (either on the same carrier particles or distinct particles) using an organic or inorganic solvent.

In certain embodiments, the composition described herein is formulated as a tablet comprising in addition to estetrol and drospirenone a filler, a superdisintegrant, a binder and disintegrant, a further binder, and a lubricant. In preferred embodiments, the composition is comprised in a tablet comprising estetrol and drospirenone, lactose, sodium starch glycolate, maize/corn starch, povidone, and magnesium stearate. Preferably, the composition described herein is formulated as a tablet comprising estetrol monohydrate, drospirenone, lactose monohydrate, sodium starch glycolate type A, maize starch, povidone K30, and magnesium stearate. Optionally, the tablet is coated with a coating agent. In further optional embodiments, the coating agent comprises hypromellose, hydroxypropylcellulose, titanium dioxide, red iron oxide, hydrogenated cottonseed oil, and talc. By means of illustration and not limitation, a suitable coating agent is AquaPolish Pink 044.08 MS. A skilled person further appreciates that any excipients present in any dosage unit such as an oral dosage unit should adhere to pharmaceutical grade industry quality standards such as Ph. Eur. and USP-NF.

By means of illustration and not limitation, an oral dosage unit comprising the composition described herein may be manufactured by a process involving wet granulation, e.g. using an aqueous solution or an organic solution, direct compression, 3D printing, or by coating carrier particles with the estetrol and drospirenone using an organic or inorganic solvent. A skilled person appreciates that a wet granulation process may suitable comprise the successive steps of: dispensing and sieving of the active ingredient(s) and excipients, blending the sieved materials in a processor, granulation, screening (i.e. further sieving) of the granules, and one or more blending steps of the sieved granules with one or more further excipients. Afterwards, if desired in view of the final dosage unit the granules may be compressed into for example a tablet, optionally involving a coating step of said tablet.

The estetrol may be comprised in the composition described herein or final dosage unit as particles. Optionally, the estetrol particles have a D(10) from about 0.5 µm to about 10 µm, preferably from about 1 µm to about 5 µm, more preferably of from about 1.5 µm to about 2.5 µm. Optionally, the estetrol particles have a D(50) of less than 20 µm or preferably less than 12 µm, more preferably from about 5 µm to about 15 µm, preferably from about 6 µm to about 12 µm, more preferably from about 7 µm to about 11 µm, , most preferably from about 8 µm to about 12 µm. Optionally, the estetrol particles have a D(90) from about 15 µm to about 50 µm, preferably from about 20 µm to about 30 µm, more preferably from about 22 µm to about 28 µm. Optionally, the estetrol particles are further granulated into larger granulates. In certain embodiments, estetrol is comprised in the composition as a multitude of larger granulates that have a volume median diameter from about 100 µm to about 4000 µm, preferably from about 200 µm to about 1000 µm, more preferably from about 200 µm to about 600 µm.

A multitude of measurement techniques are available for determining particle-size distribution values and include sieve analysis, air elutriation analysis, photo analysis, optical counting, electro resistance counting, sedimentation, laser diffraction, laser obscuration, time of transition, acoustic spectroscopy, ultrasound attenuation microscopy, by means of a cascade impactor, or any combination thereof. Unless explicitly mentioned otherwise, the particle-size distribution values of the present disclosure are obtained by laser diffraction analysis. Laser diffraction analysis, interchangeably annotated in the art by laser diffraction spectroscopy, is a particle measurement technology based on interpretation of laser diffraction patterns passed through an object. Laser diffraction is capable to measure the geometrical dimensions of a particle. Laser diffraction protocols have been described in detail in the art on numerous occasions (e.g. as reviewed in detail in a context of particle analysis in Eshel et al., Soil Science Society of America Journal, 2004).

As progestin, drospirenone is preferred, and particularly from 0.5 mg to 10 mg of drospirenone, more particularly 1 mg to 4 mg of drospirenone, more particularly about 3 mg of drospirenone is highly preferred in the context of the present invention.

A further aspect of the invention is directed to packaging units comprising the composition described herein. The packaging units may comprise at least 14, preferably at least 21, even more preferably at least 28, containers for holding separately packaged and individually removable composition described herein, wherein each container comprises at least one composition described herein. Optionally, each of the containers for holding one or more compositions described herein is individually visually arranged to present a recommended order of administration.

The skilled person appreciates that within the scope of the present invention, each packaging unit, e.g. blister pack, may be numbered or otherwise marked. The packaging units may be provided in any suitable packaging means known in the art, non-limiting examples being troches, sachets, pouches, bottles, films, sprays, microcapsules, implants, rods or blister packs.

By means of illustration and not limitation, each packaging unit may be a sealed blister pack with a cardboard, paperboard, foil plastic backing and enclosed in a suitable cover. Each supplied plastic blister may contain 24 active tablets, that may be pink and 4 inactive tablets, that may be white.

Also envisaged in any one of the aspects defined herein are packaging units such as bottles. The material of the bottle is not particularly limiting. In preferred embodiments, the bottle is a glass bottle characterized by a color capable of reducing or preventing degradation of the contents of the bottle by e.g. UV light while maintaining a degree of transparency that allows for visual inspection of the contents of said bottle. Suitable colors include without limitation amber, cobalt, or vintage green.

In a particular embodiment of the invention the packaging unit comprises 28 containers or a multiple of 28 containers, such as 3 or six multiples of 28 containers. In a preferred embodiment, the composition described herein can be supplied in (plastic) blister packs, each containing 24 active (comprising the composition described herein - typically coloured) tablets and 4 inactive (not containing the composition described herein - typically white or colourless) tablets. Both the pink and white tablets can have a drop-shaped logo on one side. In a preferred embodiment, the composition described herein is supplied in cardboard cartons containing 1, 3 or up to 6 blister packs.

The packaging unit disclosed herein may be a "compliance package". As known from the art, "compliance packages" are packaging units of variable sizes and formats that, next to providing a suitable storage means for one or more medicaments, aim to provide assistance and/or guidance to a subject to comply with the intended periodical administration (Peck Gossel, Packaging the Pill, Manifesting Medicine: Bodies and Machines, New York: Taylor & Francis, 1999). As a non-limiting example, the packaging unit may be provided with numerical indications and/or symbols that allow the subject to keep track of for example said subject's menstrual cycle. In an alternative non-limiting example, the packaging unit may comprise means to send an electronic signal to a subject when a predefined time of administration (i.e. a certain day) is reached and the dosage unit for that time point is still contained in the packaging unit. In those examples, the electronic signal may be sent to a data storage means and/or sent to a user-defined electronic device, smartphones and smart wear begin illustrative examples hereof. In certain embodiments, distinct portions of the packaging unit(s) provide a different sensory trigger to a subject, non-limiting examples being distinct colors or roughness.

While the present specification focusses mainly on compositions comprising both estetrol and drospirenone, it is evident that estetrol-only tablets are equally envisaged. A skilled person appreciates that such estetrol-only tablets may be used in a combination treatment with a separate composition containing drospirenone, ultimately achieving the same technical effects as outlined above. Hence, in certain embodiments a composition comprising estetrol is administered at a daily amount of from about 1 mg to about 40 mg of estetrol, estetrol monohydrate, or an ester of estetrol, or the composition comprises from about 1 mg to about 40 mg estetrol, estetrol monohydrate, or an ester of estetrol. In further embodiments, the composition is administered at a daily amount of from about 5 mg to about 25 mg of estetrol, estetrol monohydrate, or an ester of estetrol, or comprises from about 5 mg to about 25 mg of estetrol, estetrol monohydrate, or an ester of estetrol. Preferably the composition the composition is administered at a daily amount of from about 10 mg to about 20 mg of estetrol, estetrol monohydrate, or an ester of estetrol, or comprises from about 10 mg to about 20 mg of estetrol, estetrol monohydrate, or an ester of estetrol. In further embodiments, the composition is administered at a daily amount of about 15 mg of estetrol, estetrol monohydrate, or an ester of estetrol, or comprises about 15 mg of estetrol, estetrol monohydrate, or an ester of estetrol. In preferred embodiments, the composition is administered at a daily amount of 14.2 mg of estetrol, or comprises 14.2 mg of estetrol. In alternative embodiments, the composition is administered at a daily amount of about 20 mg of estetrol, estetrol monohydrate, or an ester of estetrol, or comprises about 20 mg of estetrol, estetrol monohydrate.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims. The herein disclosed aspects and embodiments of the invention are further supported by the following non-limiting examples. The following specific experimental examples are provided in support of the claimed invention but are not to be seen as limiting the scope of the invention.

### EXAMPLES

### Example 1: A phase III study to evaluate safety, compliance and pharmacokinetics of estetrol monohydrate/drospirenone 15/3 mg in post-menarchal female adolescents

### Brief summary

To evaluate the safety, compliance, and pharmacokinetics profile of estetrol monohydrate (E4) 15 mg combined with drospirenone (DRSP) 3 mg in post-menarchal participants between the age of 12 and 17 years + 2 months.

### Intervention

### Drug: E4/DRSP 15/3 mg combined tablet

One E4/DRSP 15/3 mg combined tablet once per day for 24 days followed by 4 days of placebo tablets; this 28-day cyclic regimen should be taken by oral ingestion for 6 consecutive cycles. The test product was packaged in 24+4 blisters (24 active and 4 placebo film-coated tablets).

### Other Names:

15 mg estetrol monohydrate/3 mg drospirenone combined oral contraceptive

### Study arms

Experimental: E4/DRSP 15/3 mg
Single treatment arm receives E4/DRSP 15/3 mg

### Outcome measures

- Number of participants with unscheduled bleeding and/or spotting episodes [Time Frame: At Cycles 1,2, 3, 4, 5, 6 (each cycle is 28 days)]
- Number of participants with absence of bleeding/spotting episodes [Time Frame: At Cycles 1,2, 3, 4, 5, 6 (each cycle is 28 days)]
- Mean number of unscheduled bleeding and/or spotting days per cycle [Time Frame: At Cycles 1,2, 3, 4, 5, 6 (each cycle is 28 days)]
- Mean number of scheduled bleeding and/or spotting days per cycle [Time Frame: At Cycles 1,2, 3, 4, 5, 6 (each cycle is 28 days)]
- Change in the Score of the Menstrual Distress Questionnaire (MDQ) from baseline to Cycles 1, 3 and 6 [Time Frame: At Baseline and at Cycles 1, 3 and 6 (each cycle is 28 days)]
   ∘ The MDQ is a standard method for measuring cyclical perimenstrual symptoms. The participants will rate common symptoms and feelings associated with menstruation using the following scale: 0 (no experience of symptom), 1 (present, mild), 2 (present, moderate), 3 (present, strong), and 4 (present, severe) observed during pre-menstrual (4 days before menstruation), menstrual (most recent flow) and intermenstrual (remainder of the cycle) phases.
- Change in Health Questionnaire for children and Young People (KIDSCREEN-27 questionnaire) from baseline to Cycles 1, 3 and 6 [Time Frame: At Baseline, Cycles 1, 3, and at the end of treatment (Cycle 6) (each cycle is 28 days)]
   ∘ KIDSCREEN-27 is a validated health-related quality of life questionnaire developed for children and adolescents. This questionnaire measures five Rasch scaled dimensions of well-being including: (1) Physical Well-being (5 items), (2) Psychological Well-being (7 items), (3) Autonomy and Parents (7 items), (4) Peers and Social Support (4 items), and (5) School Environment (4 items). Responses are indicated using a 5-point ordinal scale with responses ranging from 'never' to 'always', 'not at all' to 'extremely', or 'poor' to 'excellent'.

### Inclusion Criteria

1. Post-menarchal female participant requesting combined oral contraceptive (COC) either for contraceptive or for therapeutic use.
2. Negative serum pregnancy test at screening and negative urine pregnancy test at enrollment.
3. Aged 12 to 17 years and 2 months (inclusive) [Estonia-specific: 15 to 17 years and 2 months (inclusive)] at the time of signing the informed consent.
4. Willing to use the investigational product for 6 consecutive cycles.
5. Good physical and mental health on the basis of medical, surgical and gynecological history, physical examination, clinical laboratory, and vital signs.
6. Body mass index (BMI) below or equal to the percentile 97 (P97) on the local pediatric BMI curves.
7. Able to fulfill the requirements of the protocol, undergo all study procedures including e-diary and questionnaires completion.
8. Having indicated the willingness to participate in the study by providing written assent.
9. Having parent(s) or legal representative(s) willing and able to provide written informed consent.

### Exclusion Criteria

1. For participants who are not using hormonal contraception at screening, a menstrual cycle length shorter than 21 days or longer than 45 days.
2. Currently using an injectable or a dermally implantable hormonal method of contraception.
3. Known hypersensitivity to any of the investigational product ingredients.
4. Currently pregnant or breastfeeding or with the intention to become pregnant during the course of the study.
5. Less than 6 weeks since last delivery/2nd trimester abortion and before spontaneous menstruation has occurred following a delivery or 2nd trimester abortion.
6. Any condition representing a contraindication / precaution to the use of COCs, including but not limited to:
   1. dyslipoproteinaemia,
   2. diabetes mellitus with vascular involvement (nephropathy, retinopathy, neuropathy, other),
   3. arterial hypertension (controlled and uncontrolled)
   4. personal or first-degree family history of deep vein thrombosis or pulmonary embolism,
   5. current or planned prolonged immobilization,
   6. known inherited or acquired hypercoagulopathies or thrombogenic mutations (e.g. Factor V Leiden mutation),
   7. current treatment with anticoagulants,
   8. presence or history of arterial thromboembolism,
   9. complicated valvular heart disease,
   10. systemic lupus erythematosus,
   11. presence or history of migraine with aura,
   12. symptomatic gallbladder disease,
   13. porphyria.
7. Within the past 6 months, undiagnosed (unexplained) abnormal vaginal bleeding, or any abnormal bleeding that could possibly recur during the study.
8. Presence or history of recurrent pelvic inflammatory disease.
9. Any clinically relevant lower genital tract infection (including gonorrhea and chlamydia infections) until successfully treated, in the opinion of the Investigator.
10. Presence or history of hepatic disease as long as liver function values have not returned to normal.
11. Renal impairment
12. Hyperkalemia or presence of conditions that predispose to hyperkalemia such as renal impairment, hepatic impairment, adrenal insufficiency and daily, long-term treatment for chronic conditions or diseases with medications that may increase serum potassium concentration (e.g. angiotensin-converting-enzyme (ACE) inhibitors, angiotensin-II receptor antagonists, potassium-sparing diuretics, potassium supplementation, heparin, aldosterone antagonist and non-steroidal anti-inflammatory drugs).
13. History of organ transplantation within 5 years before screening or chronic disease potentially necessitating organ transplantation during the anticipated course of the study.
14. Presence or history of sex hormone-related malignancy.
15. History of non-hormone-related malignancy within 5 years before screening.
16. Current regular use or regular use within 1 month prior to Visit 2 of drugs potentially triggering interactions with COCs including but not limited to:
   1. Cytochrome P450 3A4 (CYP 3A4) inducers: barbiturates, primidone, bosentan, felbamate, griseofulvin, oxcarbazepine, topiramate, carbamazepine, phenytoin, rifampicin, St John's wort (Hypericum perforatum L).
   2. CYP 3A4 inhibitors: azole antifungals excluding topical fluconazole, phenylbutazone, modafinil, cimetidine, verapamil, macrolides excluding azithromycin, diltiazem and grapefruit juice.
   3. Human immunodeficiency virus (HIV)/Hepatitis C virus (HCV) protease inhibitors and non-nucleoside reverse transcriptase inhibitors.
17. History of alcohol or drug abuse (including laxatives) within 12 months prior to screening.
18. Any surgical or medical condition which might significantly alter the absorption, distribution, metabolism, or excretion of drugs.
19. Uncontrolled thyroid disorders.
20. Participation in another investigational drug clinical study within 1 month (30 days) or have received an investigational drug within the last month (30 days) prior to screening.
21. Sponsor, Contract Research Organization (CRO) or Investigator's site personnel directly affiliated with this study.
22. The participant is judged by the Investigator to be unsuitable for any reason.
23. [Estonia-specific: Subjects who are at a gynecologist visit for the first time in their life may not sign the informed consent during this first visit.]

### Key Demographics

All subjects in this study were female. The majority of subjects (93.4%) were white, 1.9% were black/ Africa American and 0.9% were Asian. Mean age was 15.2 years, ranging from 12 to 17 years. Most subjects (78.3%) were aged 15 to 17 years. Mean BMI was 21.2 kg/m2, 2.8% of subjects were in the <5th BMI percentile (underweight) and 4.7% were in the ≥ 95th BMI percentile (obese). Most of the subjects (91.5%) were starters, defined as not having used hormonal contraceptives for 2 months before screening, and 19.8% of subjects had previously used contraceptive methods such as COC's, progestin-only pills or intrauterine devices.

### Methodology

The study included 6 visits: Visit 1 (Screening Visit), Visit 2 (Subject Enrollment Visit), three on-treatment visits (Visit 3, Visit 4, Visit 5/Early Termination (ET) Visit) and one post-treatment visit (Visit 6). The site also completed an Eligibility Confirmation phone call to the subject after Visit 1 and a follow-up call after Visit 2, within 7 days following the first Investigational Product intake.

Bleeding and spotting data was summarized by the methods suggested by Mishell et al (Mishell D.R., Jr., Guillebaud J., Westhoff C., Nelson A.L., Kaunitz A.M., Trussell J., Davis A.J. Recommendations for standardization of data collection and analysis of bleeding in combined hormone contraceptive trials. Contraception. 2007;75:11-15. doi: 10.1016/j.contraception.2006.08.012.) and adapted for a COC given in a 24/4-day regimen (Table 1). Subjects recorded their daily tablet intake and absence or occurrence of vaginal bleeding/spotting event(s) in an electronic diary (e-diary) daily starting from the first day of the pre-treatment cycle. Bleeding patterns were assessed based on the response values entered by the subject in the e-diary daily.

**Table 1: Bleeding and Spotting Definitions**

| **Term** | **Definition** |
|---|---|
| Vaginal bleeding | Evidence of vaginal blood loss that requires the use of sanitary protection with a tampon, menstrual cup, pad or pantyliner. |
| Vaginal spotting | Evidence of minimal vaginal blood loss that does not require new use of sanitary protection, including pantyliners. |
| Episode of vaginal bleeding/vaginal spotting | Bleeding/spotting days bounded on either end by 2 days of no bleeding or spotting. |
| Scheduled vaginal bleeding/vaginal spotting episode | The episode has a start date within Day 25 of the cycle and Day 3 of next cycle or starts before Day 25 and stops before Day 3 of next cycle. A scheduled vaginal bleeding/vaginal spotting episode has therefore: |
| | • A start date and stop date in the scheduled bleeding interval (Day 25 - Day 3 next cycle) OR |
| | • A start date before Day 25 and stop date before Day 3 (Early withdrawal Bleeding/Spotting) OR |
| | • A start date in the scheduled bleeding interval (Day 25 - Day 3 next cycle) and continuing into next cycle (Continued withdrawal Bleeding/Spotting) |
| Unscheduled vaginal bleeding/vaginal spotting episode | The episode is defined as unscheduled if it does not meet the definition for scheduled vaginal bleeding/spotting. |

### Use of the E4/DRSP in Case of Bleeding/Spotting Episode

Scheduled bleeding was expected to begin within a few days after taking the last active tablet. If spotting/bleeding occurred while on the usual regimen of one tablet daily, the subject was advised to continue medication without interruption. If the bleeding was persistent or prolonged, participants were instructed to call the Investigator who would decide on the best management for the subject. The bleeding profile was recorded by the subject on the e-diary.

### Subject Diary

Subjects were instructed by the site personnel how to download and complete the e-diary mobile application during Visit 1, this was repeated during Visit 2.

The following information was to be recorded in the e-diary
Date of the first tablet intake at each cycle;
Tablet intake (number of active or placebo tablet(s) daily;
Absence or occurrence of vaginal bleeding/spotting event(s) daily, starting from the first day of the pre-treatment cycle:
   - 0 = Absence of vaginal bleeding or spotting;
   - 1 = Vaginal spotting: evidence of minimal vaginal blood loss that does not require new use of sanitary protection, including partyliners;
   - 2 = Vaginal bleeding: evidence of vaginal blood loss that requires the use of sanitary protection with a tampon, menstrual cup, pad or pantyliner;
Result of the urine pregnancy test(s) performed at home (before the first tablet intake of each new blister and in case of missing menstrual period);
Visual Analog Scale score, abbreviated as "VAS score" and questionnaires (KIDSCREEN-27 and MDQ Form C);
Use of rescue medication for dysmenorrheal pain.
At Visits 3, 4, 5, 6 and at the ET Visit (if any), the subject e-diary was reviewed by the Investigator. At Visit 6 or at ET Visit access to the e-diary was revoked.

### Results

### Cycle Control and Bleeding Pattern

### Vaginal Bleeding and/or Spotting Over Time

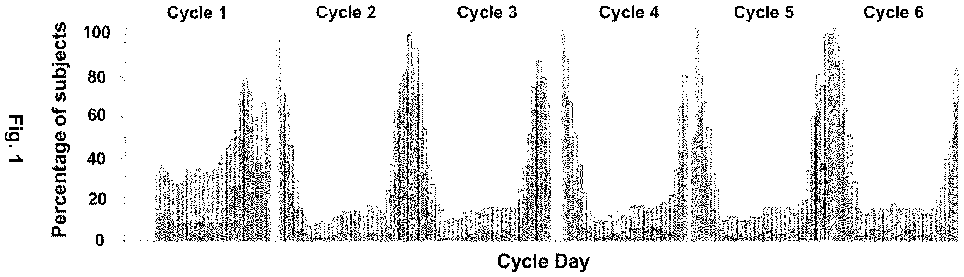

The percentage of subjects in the Modified Intent-To-Treat (mITT) population ¹ with any type of vaginal bleeding/spotting by treatment day reduced over time as shown graphically in Figure 1. Cycle 1 depicts a higher percentage of subjects reporting bleeding and/or spotting. The pattern of bleeding and/or spotting in Cycle 2 and beyond shows fewer subjects reporting bleeding and/or spotting.

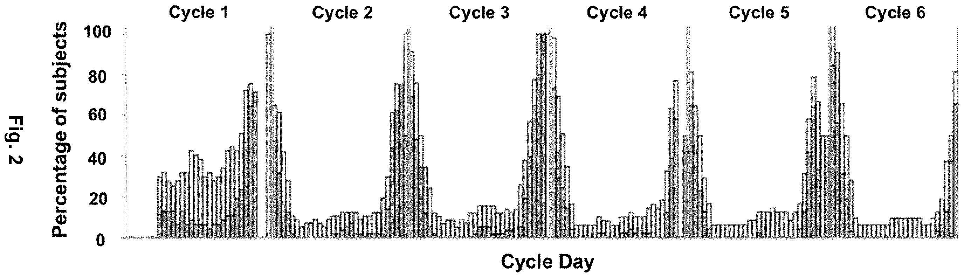

Similar results were observed in the Per-Protocol (PP) population ² (Figure 2).
¹ Modified Intent-to-treat (mITT) analysis set: subjects from the Enrolled set who received at least one dose of the study treatment, had at least one evaluable cycle, and had at least one post-baseline efficacy assessment (e-diary bleeding pattern, VAS pain questionnaire and use of rescue medication). Cycles are considered evaluable if no more than two consecutive days with missing bleeding information occurred within the cycle. One or two consecutive days with missing bleeding information are imputed with the bleeding information from the previous day.
² PP analysis set: subjects from mITT population who had no major protocol deviations that impacted the bleeding analysis.

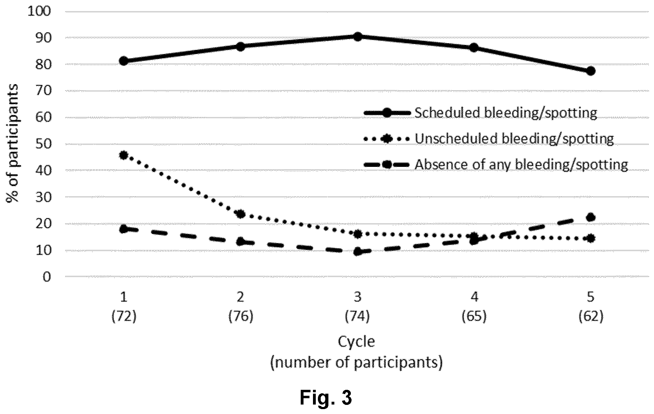

The adolescent participants showed good cycle control and acceptable bleeding pattern (Figure 3).

### Unscheduled Bleeding and/or Spotting

The number and percentage of subjects with unscheduled bleeding and/or spotting by cycle are summarized for the mITT population including evaluable cycles only (Table 2) and for the PP population (Table 3).

**Table 2: Number and percentage of subjects with unscheduled bleeding and/or spotting episodes by cycle - frequency table, Evaluable Cycles Only (mITT Population)**

| **Cycle Name** | **Number of Subjects** | **Statistic** | **E4/DRSP 15/3 mg (N=84)** |
|---|---|---|---|
| Spotting only | | | |
| Cycle 1 | 72 | n (%) | 14 (19.4%) |
| Cycle 2 | 76 | n (%) | 8 (10.5%) |
| Cycle 3 | 74 | n (%) | 8 (10.8%) |
| Cycle 4 | 65 | n (%) | 4 (6.2%) |
| Cycle 5 | 62 | n (%) | 3 (4.8%) |

| Bleeding and/or spotting | | | |
|---|---|---|---|
| Cycle 1 | 72 | n (%) | 33 (45.8%) |
| Cycle 2 | 76 | n (%) | 18 (23.7%) |
| Cycle 3 | 74 | n (%) | 12 (16.2%) |
| Cycle 4 | 65 | n (%) | 10 (15.4%) |
| Cycle 5 | 62 | n (%) | 9 (14.5%) |

Percentages are based on the number of evaluable subjects in each cycle. Only evaluable cycles are used.

**Table 3: Number and percentage of subjects with unscheduled bleeding and/or spotting episodes by cycle - frequency table (Per Protocol Population)**

| **Cycle Name** | **Number of Subjects** | **Statistic** | **E4/DRSP 15/3 mg (N=78)** |
|---|---|---|---|
| Bleeding and spotting | | | |
| Cycle 1 | 47 | n (%) | 10 (21.3%) |
| Cycle 2 | 57 | n (%) | 6 (10.5%) |
| Cycle 3 | 58 | n (%) | 2 (3.4%) |
| Cycle 4 | 49 | n (%) | 3 (6.1%) |
| Cycle 5 | 48 | n (%) | 2 (4.2%) |

| Bleeding only | | | |
|---|---|---|---|
| Cycle 1 | 47 | n (%) | 0 |
| Cycle 2 | 57 | n (%) | 0 |
| Cycle 3 | 58 | n (%) | 0 |
| Cycle 4 | 49 | n (%) | 1 (2.0%) |
| Cycle 5 | 48 | n (%) | 0 |

Percentages are based on the number of evaluable subjects in each cycle.

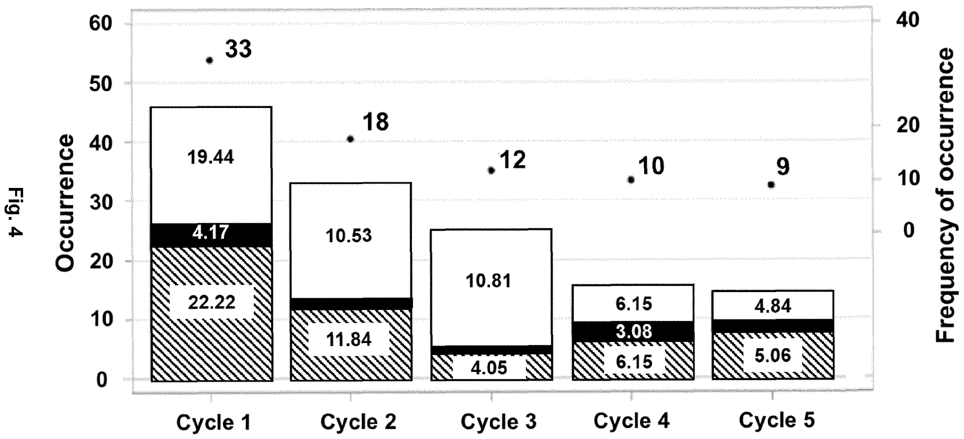

The number and percentages of subjects in the mITT population with unscheduled bleeding and/or spotting is summarized in Table 4 and are presented in Figure 4.

The percentage of subjects with unscheduled bleeding and/or spotting per cycle decreased over the study duration, from 45.8% in Cycle 1 to 14.5% in Cycle 5.

The median number of days with unscheduled bleeding and/or spotting decreased from 9 days in Cycle 1 to between 4 and 6 days over Cycles 2 to 5.

**Table 4: Percentage of Subjects and Number of Days with Unscheduled Bleeding and/or Spotting Episodes by Cycle (mITT Population, Evaluable Cycles only)**

| **Cycle Name** | **Number of Subjects** | **Incidence of Unscheduled Bleeding and/or Spotting [n(%)]** | **Number of Days with Unscheduled Bleeding and/or Spotting. Median** |
|---|---|---|---|
| Cycle 1 | 72 | 33(45.8) | 9 |
| Cycle 2 | 76 | 18 (23.7) | 5 |
| Cycle 3 | 74 | 12 (16.2) | 5 |
| Cycle 4 | 65 | 10 (15.4) | 4 |
| Cycle 5 | 62 | 9 (14.5) | 6 |

Percentages are based on the number of evaluable subjects in each cycle. Only evaluable cycles are used.

A large proportion of unscheduled bleeding and/or spotting episodes consisted of spotting only. The reduction over time was predominantly due to a reduction in episodes in which bleeding (with or without spotting) occurred (Figure 4).

Adolescents treated with E4/DRSP 15/3 mg thus showed good cycle control and acceptable bleeding pattern based on the gradual reduction in the incidence of unscheduled bleeding/spotting from 45.8% to 14.5% and a reduction in the number of unscheduled bleeding and/or spotting days per cycle from 9 to 6 days.

## Claims

1. A composition comprising an estetrol component and drospirenone for use in treating unscheduled bleeding and/or spotting in an adolescent female subject, preferably for decreasing unscheduled bleeding events and/or spotting events.

2. The composition for use according to claim 1, wherein a bleeding event is any evidence of vaginal blood loss that requires the use of sanitary protection with a tampon, menstrual cup, pad or pantyliner and wherein a spotting event is any evidence of minimal vaginal blood loss that does not require new use of sanitary protection, including panty liners.

3. The composition for use according to claim 1 or 2, wherein an unscheduled bleeding event or spotting event is a bleeding or spotting event that is not considered a scheduled bleeding event or spotting event, preferably wherein a scheduled bleeding event or spotting event has:
- a start date and stop date in the scheduled bleeding interval (day 25 - day 3 next cycle); or
- a start date before day 25 and stop date before day 3 (early withdrawal bleeding/spotting); or
- a start date in the scheduled bleeding interval (day 25 - day 3 next cycle) and continuing into next cycle (continued withdrawal bleeding/spotting).

4. The composition for use according to any one of claims 1 to 3, wherein the unscheduled bleeding events and/or spotting events are decreased compared to the unscheduled bleeding events and/or spotting events prior to the use of said composition.

5. The composition for use according to any one of claims 1 to 4, wherein the decrease in the unscheduled bleeding events and/or spotting events prior to the use of the composition is a decrease in the unscheduled bleeding and/or spotting compared to the unscheduled bleeding events and/or spotting events upon using an oral contraceptive, preferably upon using a progestin-only pill preferably comprising drospirenone, preferably upon using a combined oral contraceptive comprising as estrogenic component a component that is not an estetrol component, preferably wherein said combined oral contraceptive comprising as estrogenic component a component that is not an estetrol component is a 2nd generation combined oral contraceptive.

6. The composition for use according to any one of claims 1 to 5, wherein the unscheduled bleeding events and/or spotting events are decreased in occurrence and/or intensity by at least about 25%, preferably at least about 50%, more preferably by at least about 75%.

7. The composition for use according to any one of claims 1 to 6, wherein the number of days said unscheduled bleeding events and/or spotting events occur is reduced to 8 days or less, preferably to 6 days or less, more preferably 5 days or less within one cycle.

8. The composition for use according to any one of claims 1 to 7, wherein the decrease in unscheduled bleeding events and/or spotting events is a decrease in the intensity of unscheduled bleeding events and/or spotting events, preferably over a period of at least one month.

9. The composition for use according to any one of claims 1 to 8, wherein the estetrol component is administered at a daily dose of from about 1 mg to about 40 mg or wherein the composition comprises from about 1 mg to about 40 mg of an estetrol component.

10. The composition for use according to any one of claims 1 to 9, wherein the estetrol component is administered at a daily dose of from about 5 mg to about 25 mg, preferably at a daily dose of from about 10 mg to about 20 mg, more preferably at a daily dose of about 15 mg or wherein the composition comprises from about 5 mg to about 25 mg of an estetrol component, preferably from about 10 mg to about 20 mg of an estetrol component, more preferably about 15 mg of an estetrol component.

11. The composition for use according to any one of claims 1 to 10, wherein the drospirenone is administered at a daily dose of from about 0.5 mg to about 10 mg or wherein the composition comprises from about 0.5 mg to about 10 mg of drospirenone.

12. The composition for use according to any one of claims 1 to 11, wherein the drospirenone is administered at a daily dose of from about 1 mg to about 4 mg or wherein the composition comprises from about 1 mg to about 4 mg of drospirenone.

13. The composition for use according to any one of claims 1 to 12, wherein the estetrol component is estretrol, preferably wherein the estetrol component is estetrol monohydrate, more preferably wherein the estetrol component is administered at a daily dose of about 15 mg estetrol monohydrate or wherein the composition comprises about 15 mg of estetrol monohydrate.

14. The composition for use according to any one of claims 1 to 13, wherein the drospirenone is administered at a daily dose of about 3 mg or wherein the composition comprises about 3 mg of drospirenone.

15. The composition for use according to any one of claims 1 to 14, wherein the estetrol component and drospirenone are formulated together in a single oral dosage unit (i.e. a Combined Oral Contraceptive - COC).
